# EUROPÄISCHE PATENTSCHRIFT

(11) **EP 0 547 444 B1**
(45) Veröffentlichungstag und Bekanntmachung des Hinweises auf die Patenterteilung: **28.06.1995**
(21) Anmeldenummer: 92120659.5
(22) Anmeldetag: 03.12.1992
(51) Int. Cl.: C07C 269/04, C07C 271/28, C07C 263/04

(54) **Verfahren zur Herstellung von hochreinen aromatischen Di- und/oder Polyurethanen**
Process for the preparation of high-purity aromatic di- and/or polyurethanes
Procédé de préparation de di- et/ou polyuréthanes aromatiques de haute pureté

(30) Priorität: 16.12.1991 DE 4141402
(43) Veröffentlichungstag der Anmeldung: 23.06.1993
(73) Patentinhaber: BAYER AG, 51368 Leverkusen (DE)
(72) Erfinder: Friederichs, Wolfgang, Dr., W-5000 Köln 41 (DE); Penninger, Stefan, Dr., W-5024 Pulheim 3 (DE); Wershofen, Stefan, Dr., W-4050 Mönchengladbach 2 (DE)

(56) Entgegenhaltungen:
- EP-A- 0 018 581
- EP-A- 0 018 583
- EP-A- 0 019 109
- EP-A- 0 028 338
- US-A- 4 369 141

## Beschreibung

Die vorliegende Erfindung betrifft ein Verfahren zur Herstellung von hochreinen aromatischen Di- und/oder Polyurethanen durch Umsetzung von aromatischen Di- und/oder Polyaminen mit unsubstituierten Carbamaten unter Freisetzung von Ammoniak.

Die Herstellung von aromatischen Di- und/oder Polyurethanen aus aromatischen Di- und/oder Polyaminen und unsubstituierten Carbamaten ist von großem Interesse, da sich aromatische Di- und/oder Polyurethane durch thermische Spaltung in aromatische Di- und/oder Polyisocyanate überführen lassen, die ihrerseits Ausgangsmaterialien zur Herstellung von hochwertigen Polyurethan-Kunststoffen sind. Da die als Reaktionspartner zur Herstellung der aromatischen Di- und/oder Polyurethane verwendeten unsubstituierten Carbamate aus Harnstoff und Alkoholen zugänglich sind, können somit aromatische Di- und/oder Polyisocyanate auf phosgenfreiem Wege hergestellt werden.

Verfahren zur Herstellung von aromatischen Di- und/oder Polyurethanen aus aromatischen Di- und/oder Polyaminen und unsubstituierten Carbamaten sind bekannt und werden in zahlreichen Patenten beschrieben (DE 2 942 511, DE 2 917 568. DE 2 943 480, EP 18 583). Die bisherigen Verfahren haben allerdings den Nachteil. daß die dabei gebildeten Di- und/oder Polyurethane nicht in reiner Form anfallen. Eine ausreichende Reinheit ist jedoch Voraussetzung für eine erfolgreiche Urethanspaltung und somit eine optimale Herstellung von aromatischen Di- und/oder Polyisocyanaten. Im allgemeinen enthalten die nach den bekannten Verfahren hergestellten Produkte Ausgangsmaterialien und/oder zwangsweise anfallende Nebenprodukte, wie z.B. Aminourethane, Harnstoffurethane, Oligo- bzw. Polyharnstoffe. Diese Verunreinigungen lassen sich nur mit großem Aufwand von den Verfahrensprodukten abtrennen, was sich negativ auf die Herstellungskosten auswirkt.

Eine Ausnahme bilden aromatische Diurethane auf Basis Methanol, wie sie bei der Herstellung aus aromatischen Diaminen und Methylcarbamat anfallen. Diese Urethane besitzen im allgemeinen einen hohen Schmelzpunkt, gute Kristallisationseigenschaften und geringe Löslichkeit in organischen Lösungsmittein. Sie fallen daher, im Gegensatz zu aromatischen Diurethanen auf Basis anderer Alkohole, als reine kristalline Feststoffe aus dem Reaktionsgemisch aus oder können verhältnismäßig einfach durch Umkristallisieren von den bei der Urethan-Spaltung störenden Verunreinigungen abgetrennt werden. Der hohe Schmelzpunkt und die geringe Löslichkeit der Diurethane auf Basis Methanol wirken sich aber nachteilig auf die Verwendung der Verfahrensprodukte zur Herstellung von Isocyanaten aus. Die Umsetzung der Verfahrensprodukte in Isocyanate erfolgt durch thermische Spaltung der zugrundeliegenden Urethane. Diese Spaltung wird technisch in einer Spaltapparatur bei Temperaturen von über 200°C durchgeführt, wobei die Urethane in geschmolzenem oder gelöstem Zustand kontinuierlich zudosiert werden (siehe z.B. US 4 388 246, US 4 081 472, DE 2 421 503, DE 2 526 193, DE 3 142 627. DE 3 108 990, DE 3 215 591). Diese Art der Förderung ist aber bei Diurethanen auf Basis Methanol nicht oder nur unter Inkaufnahme von großen Nachteilen möglich. da deren hoher Schmelzpunkt über dem Zersetzungspunkt liegt und die schlechte Löslichkeit hohe Verdünnungsgrade erforderlich macht, was die Raum-Zeit-Ausbeute verschlechtert und höhere Kosten bei der destillativen Lösungsmittelrückgewinnung bewirkt.

Aromatische Di- und/oder Polyurethane werden z.B. gemäß der Lehre von DE 2 917 568 und EP 18 583 aus den bei der Umsetzung von aromatischen Di- und/oder Polyaminen mit Carbamaten vorliegenden Rohgemischen isoliert, indem der gegebenenfalls mitverwendete Katalysator abgetrennt und die gegebenenfalls vorliegenden Festprodukte abfiltriert werden. Danach wird der Alkohol und/oder das Lösungsmittel sowie das gegebenenfalls im Überschuß eingesetzte Carbamat vollständig bzw. partiell abdestilliert und das Produkt durch Auskristallisieren, Ausfällen mit oder auch durch Umkristallisieren aus anderen Lösungsmitteln gewonnen. So wird in EP 18 583, Beispiel 36, 2,4-Bis(ethoxycarbonylamino)toluol gereinigt, indem man den als Lösungsmittel verwendeten Alkohol und überschüssiges Ethylcarbamat unter vermindertem Druck bei 10 mbar ahdestilliert, den Rückstand in Methylenchlorid auflöst und ihn mehrmals mit Wasser wascht. Danach wird Methylenchlorid abgetrennt, Ethanol hinzugegeben und das Gemisch in einer Eis-Kochsalz-Mischung abgekühlt. Dabei kristallisiert 2,4-Bis(ethoxycarbonylamino)toluol mit einem Schmelzpunkt von 108 bis 110°C aus, ist jedoch wegen seiner ungenügenden Reinheit nicht für die Urethan-Spaltung geeignet. Analysenreines 2,4-Bis(ethoxycarbonylamino)toluol besitzt einen Schmelzpunkt von 132°C.

Aufgabe der vorliegenden Erfindung war es daher, ein Verfahren zur Herstellung von hochreinen aromatischen Di- und/oder Polyurethanen zur Verfügung zu stellen, die für die Herstellung von aromatischen Di- und/oder Polyisocyanaten durch thermische Urethanspaltung geeignet sind.

Es wurde nun gefunden, daß man hochreine aromatische Di- und/oder Polyurethane dadurch erhält, daß man die nach dem Stand der Technik bekannte Reaktion von aromatischen Di- und/oder Polyaminen mit unsubstituierten Carbamaten unter Freisetzung von Ammoniak so durchführt, daß die Carbamate im Überschuß eingesetzt werden, nach beendeter Reaktion das gegebenenfalls verwendete Lösungsmittel abgetrennt und das verbleibende Produktgemisch mit Wasser extrahiert wird.

Gegenstand der Erfindung ist ein Verfahren zur Herstellung von hochreinen aromatischen Di- und/oder Polyurethanen durch Umsetzung der entsprechenden aromatischen Di- und/oder Polyamine mit unsubstituierten Carbamaten unter Freisetzung von Ammoniak, welches dadurch gekennzeichnet ist, daß die Umsetzung in Gegenwart von überschüssigem Carbamat durchgeführt wird, das nach der Umsetzung vorliegende Reaktionsgemisch vom gegebenenfalls verwendeten Lösungsmittel befreit wird und die gebildeten aromatischen Di- und/oder Polyurethane durch Extraktion mit Wasser von Nebenprodukten, überschüssigem Carbamat und nicht umgesetzten Di- und/oder Polyaminen befreit werden.

Bevorzugt werden pro Mol Aminogruppe in den aromatischen Di- und/oder Polyaminen 2 bis 50 Mol, vorzugsweise 10 bis 30 Mol Carbamat eingesetzt.

Als bevorzugte Carbamate werden Ethyl-, Propyl-, Isopropylcarbamat oder deren Gemische eingesetzt.

In einer besonderen Ausführungsform werden ungereinigte Carbamate, wie sie bei der Umsetzung von Harnstoff mit dem entsprechenden Alkoholen anfallen, eingesetzt.

Die Umsetzung der entsprechenden aromatischen Di- und/oder Polyamine mit unsubstituiertem Carbamaten erfolgt vorzugsweise mit in situ hergestellten Carbamaten, indem Harnstoff und entsprechender Alkohol bei der Umsetzung eingesetzt werden.

Bei der Umsetzung gegebenenfalls verwendetes Lösungsmittel wird vorzugsweise durch Destillation entfernt.

In einer bevorzugten Ausführungsform des Verfahrens wird das erhaltene Reaktionsgemisch gegebenenfalls nach Abtrennen des Lösungsmittels extrahiert, indem das Gemisch bei 20 bis 200°C, vorzugsweise bei 50 bis 100°C mit Wasser im Verhältnis von Gemisch zu Wasser von 0,2 bis 10, vorzugsweise 0,8 bis 2 intensiv vermischt wild, anschließend auf Temperaturen von 0 bis 100°C, vorzugsweise 20 bis 70°C abgekühlt wird und die als flüssige oder feste Phase anfallenden Di- und/oder Polyurethane abgetrennt werden.

Die nach der Extraktion anfallenden aromatischen Di- und/oder Polyurethane werden vorzugsweise getrocknet und/oder zusätzlich aus organischen Lösungsmitteln. vorzugsweise aromatischen Kohlenwasserstoffen, besonders bevorzugt Benzol, Toluol, Xylol oder deren Gemische gegebenenfalls in Anwesenheit von oberflächenaktiven Stoffen, insbesondere Aktivkohle, Bleicherde, Aluminiumoxid, Alumosilikat oder Zeolith umkristallisiert.

In einer besonderen Ausführungsform des Verfahrens wird die nach der Extraktion anfallende wäßrige Phase, gegebenenfalls unter Rühren auf Temperaturen von -5 bis 20°C abgekühlt und das sich abscheidende, hauptsächlich aus Carbamat, Aminourethanen, Harnstoffurethanen, Oligo- und Polyharnstoffen, gegebenenfalls Di- und/oder Polyaminen und Di- und/oder Polyurethanen bestehende Gemisch nach Abtrennung und Trocknung gegebenenfalls zusammen mit Alkoholen in die Umsetzung zurückgeführt wird.

Insbesondere kann auch die nach Abtrennung des Gemisches als Mutterlauge anfallende wäßrige Phase, gegebenenfalls zusammen mit Frischwasser zur Extraktion eingesetzt werden.

Ein weiterer Gegenstand der Erfindung ist die Verwendung der nach dem erfindungsgemäßen Verfahren erhaltenen aromatischen Di- und/oder Polyurethane als Ausgangsmaterial zur Herstellung von Di- und/oder Polyisocyanaten.

Die Effizienz der Reinigung der Reaktionsprodukte durch Extraktion mit Wasser ist überraschend, da nicht erwartet werden konnte, daß sich organische Verunreinigungen mit Wasser extrahieren lassen, zumal die Extraktion der Reaktionsprodukte mit Wasser gemäß EP 19853, Beispiel 36 nicht den gewünschten Reinigungserfolg brachte.

Das erfindungsgemäße Verfahren besitzt den Vorteil, daß z.B. Ethylcarbamat, nicht umgesetztes Ausgangsamin und die Nebenprodukte auf einfache und schonende Weise vom erfindungsgemäßen Produkt abgetrennt werden können und die aromatischen Di- und/oder Polyurethane in hoher Reinheit anfallen.

Das erfindungsgemäße Verfahren besitzt darüber hinaus den Vorteil, daß die in der wäßrigen Phase vorliegenden, nicht umgesetzten Ausgangsamine, Nebenprodukte und die überschüssigen Carbamate zusammen in einem Verfahrensschritt aus dem Wasser abgetrennt werden können und unmittelbar oder gegebenenfalls nach separater Trocknung gegebenenfalls zusammen mit Alkoholen zur Umsetzung zurückgeführt werden können. Nebenprodukte können also ohne großen technischen Aufwand abgetrennt und durch die Rückführung nochmals der Umsetzung ausgesetzt werden.

Bei den Nebenprodukten handelt es sich, wie oben erwähnt, hauptsächlich um Aminourethane, Harnstoffurethane, Oligo- und Polyharnstoffe, das heißt, es liegen Aminoaromaten und Harnstoffverbindungen vor. Harnstoffderivate reagieren bekanntlich mit Alkoholen zu Urethanen und Aminoaromaten, letztere können mit Carbamaten unter Freisetzung von Ammoniak in Urethane überführt werden.

Das erfindungsgemäße Verfahren ist geeignet zur Herstellung von hochreinen, aromatischen Di- und/oder Polyurethanen der allgemeinen Formel:

R¹-[-NH-CO-OR²]ₙ

wobei
- R¹: für einen, gegebenenfalls substituierten, aromatischen Kohlenwasserstoffrest mit 5 bis 18 Kohlenstoffatomen oder gegebenenfalls substituiertes Diphenylmethan und/oder gegebenenfalls substituiertes, mit Methylengruppen verknüpftes polymeres Diphenylmethan
- R²: für einen Alkylrest mit 2 bzw. 3 Kohlenstoffatomen und
- n: für eine ganze Zahl größer 1
steht.

Als Substituenten für den aromatischen Kohlenwasserstoffrest R¹ kommen Alkylgruppen mit 1 bis 12 Kohlenstoffatomen in Betracht.

Bevorzugte Beispiele der aromatischen Di- und/oder Polyurethane sind O-Ethyl-, O-Propyl- und O-Isopropylurethane auf Basis verschiedener Di- und/oder Polyamine bzw. Gemische von Di- und/oder Triaminen wie z.B. m-Phenylen-, p-Phenylen-, 1,5-Naphthylen-, 2,7-Naphthylen- und 2,4-Toluylendiamin sowie Gemische von 2,4- und 2,6-Toluylendiamin, von 2,2'-, 2,4'- und 4,4'-Diaminodiphenylmethan, Polyphenylen-polymethylen-polyamine und deren Gemische, Isomerengemische von Methyl(diaminodiphenylmethan) und Methyl(triaminodiphenylmethan) und deren Gemische.

Ausgangsmaterialien für das erfindungsgemäße Verfahren sind primäre Amine der allgemeinen Formel

R¹(NH₂)ₙ

in welcher R¹ und n die oben genannte Bedeutung haben.

Die nachfolgend aufgeführten Di- und/oder Polyamine sind besonders bevorzugt geeignet:
m-Phenylendiamin. p-Phenylendiamin, 1,5- und 2,7-Diaminonaphthalin, 2,4-Toluylendiamin, 2,6-Toluylendiamin und deren Gemische, 2,2'-, 2,4'- und 4,4'-Diaminodiphenylmethan (MDA) und deren Gemische, Gemische von Diaminodiphenylmethan mit Polyphenyl-polymethylen-polyaminen ("Polymer-MDA"), Isomerengemische von Diaminomethyldiphenylmethan, Triaminomethyldiphenylmethan und deren Gemische.

Als Carbamate kommen Ethyl-, Propyl- oder Isopropylcarbamat zum Einsatz. Sie werden vorzugsweise in reiner Form eingesetzt. Selbstverständlich können auch ungereinigte Carbamate, wie sie bei der Umsetzung von Harnstoff mit Alkoholen anfallen, eingesetzt werden. Außerdem ist es möglich die Carbamate in situ aus Harnstoff und Alkoholen zu erzeugen.

Als Lösungsmittel werden gegebenenfalls organische Lösungsmittel eingesetzt. Im bevorzugten Fall, wenn die Reaktion unter Druck durchgeführt wird, werden Alkohole, insbesondere die den eingesetzten Carbamaten zugrundeliegenden Alkohole mit 2 bzw. 3 Kohlenstoffatomen verwendet. Falls jedoch die Reaktion unter Normaldruck durchgeführt wird, werden hochsiedende, vorzugsweise aprotisch polare Lösungsmittel wie z.B. Dimethylformamid oder Dimethylacetamid eingesetzt.

Die Reaktion wird im allgemeinen ohne Katalysatoren durchgeführt, sie kann aber auch mit den Katalysatoren des Stands der Technik beschleunigt werden.

Der bei der Reaktion vorliegende Druck ist abhängig von der Reaktionstemperatur und von der Verfahrensweise. Die Reaktion kann bei Normaldruck oder unter erhöhtem Druck, vorzugsweise Eigendruck, kontinuierlich oder diskontinuierlich durchgeführt werden.

Die Reaktion wird bevorzugt bei erhöhten Temperaturen im Temperaturbereich von 120 bis 350°C. besonders bevorzugt bei Temperaturen von 180 bis 220°C durchgeführt.

Bei der Durchführung der Reaktion werden aromatische Di- und/oder Polyamine mit Carbamaten, gegebenenfalls in Anwesenheit von Lösungsmitteln, bevorzugt im Molverhältnis (Aminogruppe : Carbamat) von 1 zu 2 bis 1 zu 50, vorzugsweise 1 zu 10 bis 1 zu 30 auf Reaktionstemperatur gebracht und Ammoniak gegebenenfalls zusammen mit dem Lösungsmittel aus dem Reaktionsgemisch abdestilliert. Wie oben aufgeführt kann das Carbamat in situ aus Harnstoff und niedrigsiedenden Alkoholen hergestellt werden, so daß die genannten Molverhältnisse auch für das System aromatisches Di- und/oder Polyamin zu Harnstoff/Alkohol gelten, d.h. pro Mol aromatischer Aminogruppe werden 2 bis 50 Mol, vorzugsweise 10 bis 30 Mol Harnstoff und 2 bis 50 Mol, vorzugsweise 10 bis 30 Mol Alkohol benötigt. Es empfiehlt sich jedoch, falls das Carbamat in situ aus Harnstoff und Alkohol hergestellt werden sollte, den Alkohol als Lösungsmittel zu verwenden.

Eine besonders bevorzugte Ausführungsform der Reaktion ist die Durchführung unter Druck, wobei als Lösungsmittel die den Carbamaten zugrundeliegenden Alkohole dienen. Das während der Reaktion gebildete Ammoniak wird zusammen mit dem Lösungsmittelalkohol abdestilliert, wobei Alkoholverluste ergänzt werden. Der erforderliche Druck wird über ein Druckregelventil eingestellt. Selbstverständlich ist es möglich, reines Ammoniak über eine Kolonne unter Druck abzudestillieren. In diesem Fall kann das Nachdosieren des Alkohols entfallen.

Der Anteil des Lösungsmittels liegt bei 5 bis 90 Gew.-%, vorzugsweise bei 10 bis 50 Gew.-%, bezogen auf den gesamten Ansatz.

Die Reaktionszeiten betragen 1 bis 20 Stunden, vorzugsweise 5 bis 10 Stunden.

Nach Beendigung der Reaktion wird das Lösungsmittel gegebenenfalls unter vermindertem Druck abdestilliert und Carbamat zusammen mit den Nebenprodukten aus dem verbleibenden Rohprodukt durch Extraktion mit Wasser abgetrennt. Dies geschieht, indem man das Rohprodukt bei Temperaturen von 20 bis 200°C; vorzugsweise bei 50 bis 100°C mit Wasser im Verhältnis (Rohprodukt zu Wasser) von 0,2 bis 10, vorzugsweise 0,8 bis 2 intensiv vermischt, anschließend das Gemisch gegebenenfalls unter Rühren auf Temperaturen von 0 bis 100°C, vorzugsweise 20 bis 70°C abkühlt und die Phasen trennt. Die Extraktion kann gegebenenfalls mehrmals wiederholt werden, wobei die Extraktionsbedingungen bezüglich Druck. Temperatur und Mischungsverhältnis variiert werden können.

Feste Verfahrensprodukte, wie z.B. aromatische Diurethane, werden von der wäßrigen Phase abfiltriert und fallen nach Trocknung als hochreine, für die thermische Urethanspaltung geeignete Urethane an. Ihre Reinheit kann, falls erwünscht, noch durch zusätzliches Umkristallisieren aus organischen Lösungsmitteln, vorzugsweise aromatischen Kohlenwasserstoffen wie z.B. Benzol, Toluol, Xylol und deren Gemischen, gegebenenfalls in Gegenwart von oberflächenaktiven Stoffen wie z.B. Aktivkohle, Bleicherde, Aluminiumoxid, Alumosilikat und Zeolit verbessert werden.

Flüssige Verfahrensprodukte wie z.B. Gemische von Di- und/oder Polyurethanen scheiden sich, bedingt durch ihre unterschiedliche Dichte, von der wäßrigen Phase ab und können somit abgetrennt werden. Ihre Reinheit kann ebenfalls durch Kontakt mit dem obengenannten oberflächenaktiven Stoffen verbessert werden.

Die Verfahrensprodukte fallen im allgemeinen als analysenreine Substanzen an.

Die Wasserphase wird gegebenenfalls unter Rühren auf Temperaturen von -5 bis 20°C abgekühlt, wobei sich ein Stoffgemisch abscheidet, das aus Carbamat, Aminourethanen, Harnstoffürethanen, Oligo- und Polyharnstoffen und gegebenenfalls geringen Mengen an Ausgangsamin und Di- und/oder Polyurethanen besteht. Das Gemisch wird je nach Aggregatzustand abfiltriert bzw. abgeschieden und kann nach Trocknung gegebenenfalls nach Zugabe von frischen aromatischen Di- und/oder Polyaminen und Carbamaten gegebenenfalls zusammen mit Alkoholen in die Umsetzung zurückgeführt werden. Dadurch kann die Ausbeute der Verfahrensprodukte erhöht werden.

Die nach der Abtrennung des Gemisches verbleibende wäßrige Mutterlauge kann ebenfalls gegebenenfalls im Gemisch mit Frischwasser oder den anderen obengenannten wäßrigen Phasen zur extraktiven Reinigung des Reaktionsprodukts verwendet werden.

Anhand der nachfolgenden Beispiele soll die Erfindung näher erläutert werden. Prozentangaben beziehen sich, falls nicht anders enwähnt, auf Gewichtsprozente.

### Beispiel 1: Herstellung von 2,4-Bis(ethoxycarbonylamino)toluol

In einem 5 l Rührautoklav werden 122 g (1 Mol) 2,4-Diaminotoluol mit 1 782 g (20 Mol) Ethylcarbamat in Gegenwart von 320 g Ethanol unter Rühren auf 200°C erwärmt und pro Stunde 1 300 ml Ammoniak-Ethanol-Gemisch abdestilliert. Mit Hilfe eines Regelventils wird ein Autoklavendruck von 6 bis 10 bar eingestellt, die Ethanolkonzentration wird durch Zupumpen von frischem Ethanol konstant gehalten. Nach 5stundiger Reaktionszeit läßt man den Autoklaven abkühlen, entleert ihn und destilliert den Alkohol bei 40 bis 90°C/90 mbar. Der Destillationsrückstand (1 224 g) wird bei 95°C in 1 200 g Wasser aufgenommen und intensiv verrührt. Nach Abkühlen der Lösung auf 40°C fallen 260 g 2,4-Bis(ethoxycarbonylamino)toluol aus, die abfiltriert, erneut in 300 g kochendem Wasser aufgenommen und wieder bei 40°C gefällt werden. Nach Trocknung im Trockenschrank bei 90°C/100 mbar erhält man 170,4 g (64,1% d.Th.) 2,4-Bis(ethoxycarbonylamino)toluol.

| Elementaranalyse: | | | | |
|---|---|---|---|---|
| C₁₃H₁₈N₂O₄ (266,30) | Ber.: | C: 58,6% | H: 6,8% | N: 10,5% |
| | Gef.: | C: 58,5% | H: 6,8% | N: 10,7% |

### Beispiel 2: Herstellung von 4,4'-Bis(ethoxycarbonylamino)diphenylmethan

In einem 5 l Rührautoklav werden 198 g (1 Mol) 4,4'-Diaminodiphenylmethan, 1 782 g (20 Mol) Ethylcarbamat und 1 200 g Ethanol unter Rühren auf 200°C erhitzt. Mit Hilfe eines Regelventils wird ein Druck von ca. 16 bar eingestellt, so daß pro Stunde 1 500 ml Ammoniak-Ethanol-Gemisch in eine mit Sole gekühlte Vorlage destillieren. Die entsprechende Ethanolmenge wird mit einer Pumpe nachdosiert. Nach 5 Stunden wird der Autoklav auf 60°C abgekühlt und entleert. Ethanol wird unter Vakuum abdestilliert. Das feste Reaktionsprodukt wird zweimal mit 1 630 g bzw. 900 g Wasser bei 60°C gewaschen, mit Toluol azeotrop entwässert und in Gegenwart von 100 g Tonsil K10®, Südchemie umkristallisiert. Nach Trocknung werden 246.0 g (71,9% der Theorie) 4,4'-Bis(ethoxycarbonylamino)diphenylmethan mit einem Schmelzpunkt von 133°C gewonnen.

| Elementaranalyse: | | | | |
|---|---|---|---|---|
| C₁₉H₂₂N₂O₄ (342,40) | Ber.: | C: 66,7% | H: 6,5% | N: 8,2% |
| | Gef.: | C: 66,8% | H: 6,5% | N: 8,2% |

Die vereinigten Waschwässer werden auf -2°C gekühlt. Dabei fallen 860 g Feststoff an, der zu 92,8 Gew.-% aus Ethylcarbamat, zu 4,4 Gew.-% aus 4,4'-Bis(ethoxycarbonylamino)diphenylmethan, zu 1,0 Gew.-% aus Aminourethan und zu 0,8 Gew.-% aus Harnstoffurethan besteht.

### Beispiel 3: Nebenprodukt- und Ethylcarbamat-Rückführung

198 g Diaminodiphenylmethan werden mit 922 g frischem Ethylcarbamat und 860 g aus Beispiel 2 gewonnenem Ethylcarbamat-haltigem Feststoff in Gegenwart von 1 200 g Ethanol wie in Beispiel 2 beschrieben zur Reaktion gebracht. Nach gleicher Aufarbeitung werden 270,0 g (78,9% d.Th.) 4,4'-Bis(ethoxycarbonylamino)diphenylmethan mit einer Reinheit von 99,4 Gew.-% isoliert.

### Beispiel 4: Herstellung von Polyphenylen-polymethylen-polyethylurethan-("Polymer-MDU")

In einem 5 l Rührautoklav werden 106,5 g Polyphenylenpolymethylenpolyamin ("Polymer-MDA"), 2 228,0 g Ethylcarbamat und 320 g Ethanol unter Rühren auf 200°C erhitzt. Mit Hilfe eines Druckregelventils wird ein Autoklavendruck von 6 bis 10 bar eingestellt, so daß ein Ammoniak-Ethanol-Gemisch mit 1 300 ml/h in eine solegekühlte Vorlage überdestilliert. Durch Zupumpen von Ethanol über eine Membranpumpe wird der Ethanolgehalt im Autoklaven konstant gehalten. Nach einer Reaktionszeit von 5 Stunden wird die Reaktion beendet, der Autoklav abgekühlt und entleert. Man entfernt den Lösungsmittelalkohol durch Destillation bei 40 bis 90°C und 90 mbar und erhält einen Rückstand von 1 308 g, der mit 1 300 g Wasser bei 95°C im Scheidetrichter extrahiert wird. Nach Phasentrennung gewinnt man 265 g Polymer-MDU, das erneut in gleicher Weise mit 250 g Wasser extrahiert wird. Das Produkt wird anschließend mit 500 ml Toluol und 30 g Tonsil K10®, Südchemie versetzt und azeotrop entwässert. Tonsil K10® wird über eine Drucknutsche abfiltriert und das Filtrat am Rotationsverdampfer eingeengt. Die Ausbeute an Polyphenylenpolymethylenpolyethylurethan ("Polymer-MDU") beträgt 126,1 g.

## Patentansprüche

1. Verfahren zur Herstellung von hochreinen aromatischen Di- und/oder Polyurethanen durch Umsetzung der entsprechenden aromatischen Di- und/oder Polyamine mit unsubstituierten Carbamaten unter Freisetzung von Ammoniak dadurch gekennzeichnet, daß die Umsetzung in Gegenwart von überschüssigen Carbamaten durchgeführt wird, daß das nach der Umsetzung vorliegende Reaktionsgemisch von gegebenenfalls verwendetem Lösungsmittel befreit und die gebildeten aromatischen Di- und/oder Polyurethane durch Extraktion mit Wasser gereinigt werden.

2. Verfahren gemäß Anspruch 1, dadurch gekennzeichnet, daß pro Mol Aminogruppe in den aromatischen Di- und/oder Polyaminen 2 bis 50 Mol, vorzugsweise 10 bis 30 Mol Carbamat eingesetzt werden.

3. Verfahren gemäß Anspruch 1, dadurch gekennzeichnet, daß als Carbamate Ethyl-, Propyl-, Isopropylcarbamat oder deren Gemische eingesetzt werden.

4. Verfahren gemäß Anspruch 1 oder 3, dadurch gekennzeichnet, daß ungereinigte Carbamate, wie sie bei der Umsetzung von Harnstoff mit den entsprechenden Alkoholen anfallen, eingesetzt werden.

5. Verfahren gemäß Anspruch 1 oder 3, dadurch gekennzeichnet daß bei der Umsetzung der entsprechenden aromatischen Di- und/oder Polyamine mit unsubstituierten Carbamaten die Carbamate in situ gebildet werden, indem Harnstoff und entsprechender Alkohol bei der Umsetzung eingesetzt werden.

6. Verfahren gemäß Anspruch 1, dadurch gekennzeichnet, daß gegebenenfalls verwendete Lösungsmittel durch Destillation abgetrennt werden.

7. Verfahren gemaß Anspruch 1, dadurch gekennzeichnet, daß gegebenenfalls nach Abtrennen des Lösungsmittels das erhaltene Reaktionsgemisch extrahiert wird, indem das Gemisch bei 20 bis 200°C, vorzugsweise bei 50 bis 100°C mit Wasser im Verhältnis von Gemisch zu Wasser von 0,2 bis 10, vorzugsweise 0,8 bis 2 intensiv vermischt wird, anschließend auf Temperaturen von 0 bis 100°C, vorzugsweise 20 bis 70°C abgekühlt wird und die als flüssige oder feste Phase anfallenden Di- und/oder Polyurethane abgetrennt werden.

8. Verfahren gemäß Anspruch 1 oder 7, dadurch gekennzeichnet, daß die nach der Extraktion anfallenden aromatischen Di- und/oder Polyurethane getrocknet und/oder zusätzlich aus organischen Lösungsmitteln, vorzugsweise aromatischen Kohlenwasserstoffen, besonders bevorzugt Benzol, Toluol, Xylol oder deren Gemische, gegebenenfalls in Anwesenheit von oberflächenaktiven Stoffen, insbesondere Aktivkohle, Bleicherde, Aluminiumoxid, Alumosilikat oder Zeolit umkristallisiert werden.

9. Verfahren gemäß Anspruch 1 oder 7, dadurch gekennzeichnet, daß die wäßrige Phase, gegebenenfalls unter Rühren auf Temperaturen von -5 bis 20°C abgekühlt wird und das sich abscheidende, aus Carbamat, Aminourethanen, Harnstoffurethanen, Oligo- und Polyharnstoffen, gegebenenfalls Di- und/oder Polyaminen und Di- und/oder Polyurethanen bestehende Gemisch nach Abtrennung und Trocknung, gegebenenfalls zusammen mit Alkoholen in die Umsetzung zurückgeführt wird.

10. Verfahren gemäß Anspruch 9, dadurch gekennzeichnet, daß die nach Abtrennung des Gemisches als Mutterlauge anfallende wäßrige Phase, gegebenenfalls zusammen mit Frischwasser zur Extraktion eingesetzt wird.

## Claims

1. Process for the production of highly pure di- and/or polyurethanes by reaction of the corresponding di- and/or polyamines with unsubstituted carbamates with liberation of ammonia, characterised in that the reaction is performed in the presence of excess carbamates, that optionally used solvents are removed from the reaction mixture obtained after the reaction and the aromatic di- and/or polyurethanes formed are purified by extraction with water.

2. Process according to claim 1, characterised in that 2 to 50 mol, preferably 10 to 30 mol of carbamate are used per mol of amino groups in the aromatic di- and/or polyamines.

3. Process according to claim 1, characterised in that ethyl, propyl, isopropyl carbamate or mixtures thereof are used as the carbamates.

4. Process according to claim 1 or 3, characterised in that unpurified carbamates, as are obtained from the reaction of urea with the corresponding alcohols, are used.

5. Process according to claim 1 or 3, characterised in that the carbamates are formed *in situ* during the reaction of the corresponding aromatic di- and/or polyamines with unsubstituted carbamates by using ureas and the corresponding alcohol in the reaction.

6. Process according to claim 1, characterised in that optionally used solvents are removed by distillation.

7. Process according to claim 1, characterised in that the reaction mixture obtained is extracted, optionally after separation of the solvent, by vigorously mixing the mixture with water at 20 to 200°C, preferably at 50 to 100°C, in a ratio of mixture to water of 0.2 to 10, preferably of 0.8 to 2 and then cooling to temperatures of 0 to 100°C, preferably of 20 to 70°C and separating the di- and/or polyurethanes obtained as a liquid or solid phase.

8. Process according to claim 1 or 7, characterised in that the di- and/or polyurethanes obtained after extraction are dried and/or additionally recrystallised from organic solvents, preferably aromatic hydrocarbons, particularly preferably benzene, toluene, xylene or mixtures thereof, optionally in the presence of surface-active substances, in particular activated carbon, bleaching earth, aluminium oxide, aluminosilicate or zeolite.

9. Process according to claim 1 or 7, characterised in that the aqueous phase is cooled to temperatures of -5 to 20°C, optionally with stirring, and the precipitated mixture of carbamate, aminourethanes, urea urethanes, oligo- and polyureas, optionally di- and/or polyamines and di- and/or polyurethanes is returned to the reaction after separation and drying, optionally together with alcohols.

10. Process according to claim 9, characterised in that the aqueous phase obtained as mother liquor after separation of the mixture is used for the extraction, optionally together with fresh water.

## Revendications

1. Procédé pour la préparation de di- et/ou polyuréthannes aromatiques à pureté élevée par mise en réaction des di- et/ou polyamines aromatiques correspondantes avec des carbamates non substitués avec libération d'ammoniac, caractérisé en ce qu'on effectue la mise en réaction en présence de carbamates en excès, qu'on libère le mélange réactionnel présent après la mise en réaction du solvant éventuellement utilisé et on purifie les di- et/ou polyuréthannes aromatiques formés par extraction dans de l'eau.

2. Procédé selon la revendication 1, caractérisé en ce que, par mole de groupe amino dans les di- et/ou polyamines aromatiques, on met en oeuvre de 2 à 50 moles, de préférence de 10 à 30 moles de carbamate.

3. Procédé selon la revendication 1, caractérisé en ce que, comme carbamates, on met en oeuvre l'éthylcarbamate, le propylcarbamate, l'isopropylcarbamate ou leurs mélanges.

4. Procédé selon la revendication 1 ou 3, caractérisé en ce qu'on met en oeuvre des carbamates non purifiés tels qu'on les obtient par la mise en réaction d'urée avec les alcools correspondants.

5. Procédé selon la revendication 1 ou 3, caractérisé en ce que, lors de la mise en réaction des di- et/ou polyamines aromatiques correspondantes avec des carbamates non substitués, on forme les carbamates in situ en mettant en oeuvre de l'urée et un alcool correspondant lors de la mise en réaction.

6. Procédé selon la revendication 1, caractérisé en ce qu'on sépare par distillation les solvants éventuellement utilisés.

7. Procédé selon la revendication 1, caractérisé en ce que, éventuellement après séparation du solvant, on extrait le mélange réactionnel obtenu en mélangeant intimement le mélange à une température de 20 à 200°C, de préférence de 50 à 100°C avec de l'eau dans un rapport du mélange à l'eau de 0,2 à 10, de préférence de 0,8 à 2, ensuite on refroidit à des températures de 0 à 100°C, de préférence de 20 à 70°C et on sépare les di- et/ou polyuréthannes que l'on obtient en phase liquide ou en phase solide.

8. Procédé selon la revendication 1 ou 7, caractérisé en ce qu'on sèche les di- et/ou polyuréthannes aromatiques que l'on obtient après l'extraction et/ou on les soumet à une cristallisation supplémentaire dans des solvants organiques, de préférence dans des hydrocarbures aromatiques, de manière particulièrement préférée dans du benzène, dans du toluène, dans du xylène ou dans leurs mélanges, éventuellement en présence de substances tensioactives, en particulier du charbon actif, l'argile décolorante, l'oxyde d'aluminium, le silicate d'aluminium ou la zéolite.

9. Procédé selon la revendication 1 ou 7, caractérisé en ce qu'on refroidit la phase aqueuse, éventuellement en agitant, à des températures de -5 à 20°C et on renvoie dans la réaction, éventuellement de manière conjointe avec des alcools, le mélange qui se dépose, constitué par du carbamate, des amino-uréthannes, des urée-uréthannes, des oligo- et poly-urées, éventuellement des di- et/ou polyamines et des di- et/ou polyuréthannes, après séparation et séchage.

10. Procédé selon la revendication 9, caractérisé en ce qu'on met en oeuvre la phase aqueuse que l'on obtient sous forme de liqueur mère après séparation du mélange, éventuellement de manière conjointe avec de l'eau fraîche, à des fins d'extraction.
